# EUROPÄISCHE PATENTSCHRIFT

(11) **EP 3 407 931 B1**
(45) Veröffentlichungstag und Bekanntmachung des Hinweises auf die Patenterteilung: **08.09.2021**
(21) Anmeldenummer: 17702596.2
(22) Anmeldetag: 27.01.2017
(51) Int. Cl.: A61M 1/16

(54) **VERFAHREN ZUM REGELN EINER HEIZVORRICHTUNG ZUM ERWÄRMEN EINES FLUIDS FÜR EINEN DIALYSIERFLÜSSIGKEITSKREISLAUF, STEUERVORRICHTUNG SOWIE BLUTBEHANDLUNGSVORRICHTUNG**
METHOD FOR REGULATING A HEATING DEVICE FOR HEATING A FLUID FOR A DIALYSIS LIQUID CIRCUIT, CONTROL DEVICE, AND BLOOD TREATMENT DEVICE
PROCÉDÉ PERMETTANT DE RÉGLER UN DISPOSITIF DE CHAUFFAGE POUR LE CHAUFFAGE D'UN FLUIDE DESTINÉ À UN CIRCUIT DE LIQUIDE DE DIALYSE, DISPOSITIF DE COMMANDE ET DISPOSITIF DE TRAITEMENT SANGUIN

(30) Priorität: 29.01.2016 DE 102016101648
(43) Veröffentlichungstag der Anmeldung: 05.12.2018
(73) Patentinhaber: Fresenius Medical Care Deutschland GmbH, 61352 Bad Homburg (DE)
(72) Erfinder: KLÖFFEL, Peter, 97720 Nüdlingen (DE); ENDER, Helmuth, 97475 Zeil am Main (DE); SCHLERETH, Michael, 97421 Schweinfurt (DE); BARDORZ, Christoph, 97228 Rottendorf (DE); EHRENBERGER, Walter, 97447 Gerolzhofen (DE)
(74) Vertreter: Bobbert & Partner Patentanwälte PartmbB
(86) Internationale Anmeldenummer: PCT/EP2017/051848
(87) Internationale Veröffentlichungsnummer: WO 2017/129791

(56) Entgegenhaltungen:
- US-A- 3 730 183
- US-A- 4 718 022
- US-A- 4 769 151

## Beschreibung

Die vorliegende Erfindung betrifft ein Verfahren zum Regeln und/oder Überwachen einer Heizvorrichtung zum Erwärmen eines Fluids gemäß Anspruch 1. Sie betrifft ferner eine Steuer- oder Regelvorrichtung gemäß Anspruch 10 sowie eine Blutbehandlungsvorrichtung gemäß Anspruch 11. Des Weiteren betrifft die vorliegende Erfindung ein digitales Speichermedium gemäß Anspruch 14 sowie, ein Computerprogramm-Produkt gemäß Anspruch 15.

Dialysierflüssigkeiten für Blutbehandlungsvorrichtungen können auf unterschiedliche Art und Weise bereitgestellt werden, bevor sie einem Dialysierflüssigkeitskreislauf zugeführt werden. Beispielsweise kann aus einem externen Wasserversorgungssystem Wasser abgeführt und für den Dialysierflüssigkeitskreislauf aufbereitet werden. Die Aufbereitung kann in einem Vorlaufkreislauf oder in einem Wassereingangssystem durchgeführt werden, in dem verschiedene Aufbereitungsschritte zur Herstellung der Dialysierflüssigkeit durchgeführt werden. Das Wasser oder die fertige Dialysierflüssigkeit wird anschließend mittels einer Heizvorrichtung erwärmt und dem Dialysierkreislauf, in welchen ein Blutfilter oder Dialysator integriert ist, zugeführt. Die US4769151 offenbart ein Verfahren zum Regeln einer Heizvorrichtung für ein Dialysatzubereitungsgerät.

Eine Aufgabe der vorliegenden Erfindung ist es, ein Verfahren zum Steuern, Regeln und/oder Überwachen einer Heizvorrichtung zum Erwärmen eines Fluids für eine Blutbehandlung anzugeben. Zudem sollen geeignete Vorrichtungen, ein geeignetes digitales Speichermedium und ein geeignetes Computerprogramm-Produkt angegeben werden.

Die erfindungsgemäße Aufgabe kann durch ein Verfahren mit den Merkmalen des Anspruchs 1 gelöst werden. Sie kann ferner gelöst werden mittels der Steuer- oder Regelvorrichtung mit den Merkmalen des Anspruchs 10 sowie mittels einer Blutbehandlungsvorrichtung mit den Merkmalen des Anspruchs 11. Sie kann zudem gelöst werden mittels des digitalen Speichermediums mit den Merkmalen des Anspruchs 14 sowie mittels des Computerprogramm-Produkts mit den Merkmalen des Anspruchs 15.

Erfindungsgemäß wird somit ein Verfahren zum Steuern oder Regeln (die Begriffe "Steuern" und "Regeln" sind hierin alternativ oder austauschbar zu verstehen; beide Modi sind erfindungsgemäß umfasst) und/oder Überwachen einer Heizvorrichtung zum Erwärmen eines Fluids vorgeschlagen, welches insbesondere über einen Zulauf in einen Vorlauf eines Dialysierflüssigkeitskreislaufs fließt oder geflossen ist. Das Fluid kann Wasser sein, das beispielsweise aus einer externen Wasserversorgungsanlage geführt wird. Der Dialysierflüssigkeitskreislauf ist Teil einer Blutbehandlungsvorrichtung oder hiermit verbunden. Der Dialysierflüssigkeitskreislauf weist einen Behälter zur Aufnahme des Fluids und einen Heizbehälter zum Erwärmen des Fluids auf, wobei der Heizbehälter in Fluidkommunikation mit dem Behälter steht.

Das Verfahren umfasst ein Starten eines Heizvorgangs zum Erwärmen des Fluids im Heizbehälter nur dann, wenn der Füllstand des Behälters mittels insbesondere direktem oder indirektem, Zuflusses einen vorbestimmten Füllstandswert (oder vorbestimmten Füllstand) wenigstens einmal, oder erstmalig, erreicht.

Manche oder alle der Schritte des erfindungsgemäßen Verfahrens können automatisiert, etwa veranlasst durch eine entsprechend programmierte oder konfigurierte, Steuervorrichtung oder Regelvorrichtung (die Begriffe "Steuervorrichtung" und "Regelvorrichtung" sind hierin alternativ oder austauschbar zu verstehen) gemäß der vorliegenden Erfindung, ablaufen. Die Steuervorrichtung kann programmiert und/oder konfiguriert sein, die Schritte des erfindungsgemäßen Verfahrens einzuleiten, auszuführen oder zu bewirken.

Die erfindungsgemäße Blutbehandlungsvorrichtung weist wenigstens eine erfindungsgemäße Steuervorrichtung auf oder ist hiermit in Signalkommunikation verbunden.

Ein erfindungsgemäßes digitales, insbesondere nicht-flüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode oder eine Signalwelle zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in manchen beispielhaften Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein offenbartes, nicht beanspruchtes Computerprogramm weist einen Programmcode auf zur Veranlassung der maschinellen Schritte des erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Für das erfindungsgemäße digitale Speichermedium, das erfindungsgemäße Computerprogramm-Produkt und das offenbarte, nicht beanspruchte Computerprogramm gilt, dass alle, einige oder manche der maschinell durchgeführten Schritte des erfindungsgemäßen Verfahrens veranlasst werden. Dies gilt insbesondere im Zusammenwirken mit einer erfindungsgemäßen Blutbehandlungsvorrichtung wie beschrieben.

Bei allen Ausführungen ist der Gebrauch des Ausdrucks "kann sein" bzw. "kann haben" usw. synonym zu "ist insbesondere" bzw. "hat insbesondere" usw. zu verstehen und/oder soll eine weitere beispielhafte, erfindungsgemäße Ausführungsform erläutern.

Erfindungsgemäße Ausführungsformen können eines oder mehrere der vorstehend oder im Folgenden genannten Merkmale in beliebiger Kombination aufweisen, sofern eine solche Kombination nicht für den Fachmann erkennbar technisch unmöglich ist. Erfindungsgemäße Ausführungsformen sind ferner auch Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

In manchen beispielhaften, erfindungsgemäßen Ausführungsformen ist der Vorlauf zum Dialysierflüssigkeitskreislauf ein Wasserkreislauf oder ein Wassereingangssystem. Der Vorlauf kann als hydraulisches System bezeichnet werden. Der Zulauf kann ein Zulauf aus einem externen Wasserversorgungssystem oder einem Wasserleitungssystem sein. Der Zulauf kann ein Wassereinlauf sein.

Im Folgenden wird der Begriff "Fluid" synonym zum Begriff "Wasser" verwendet, ohne dass das Fluid hierdurch jedoch auf Wasser beschränkt werden soll. Das Fluid kann ebenso ein anderes Medium und insbesondere eine andere Flüssigkeit sein, sofern für einen Vorlauf zum Dialysierflüssigkeitskreislauf geeignet.

In manchen beispielhaften, erfindungsgemäßen Ausführungsformen wird der vorbestimmte Füllstandswert erreicht, wenn sich der Füllstand von einem Füllstand, der einem unterhalb des vorbestimmten Füllstandwerts liegenden Füllstandswert entspricht, bei welchem der Behälter vergleichsweise leer ist, zu einem Füllstand übergeht, bei dem der Behälter vergleichsweise voll ist und dem ein auf oder oberhalb des vorbestimmten Füllstandswert liegender Füllstandswert entspricht.

In manchen beispielhaften, erfindungsgemäßen Ausführungsformen ist der vorbestimmte Füllstandswert ein Grenzwert.

In manchen beispielhaften, erfindungsgemäßen Ausführungsformen gibt der Füllstandswert, wenn er von "unten kommend" erreicht ist, an, dass der Behälter vollständig befüllt ist. Alternativ wird ungeachtet des tatsächlich Füllstandes des Behälters davon ausgegangen, dass der Behälter bei Erreichen des vorbestimmten Füllstandswerts ausreichend oder vollständig befüllt ist.

In manchen beispielhaften, erfindungsgemäßen Ausführungsformen gibt der Füllstandswert, wenn er von "oben kommend" erreicht ist, an, dass der Behälter leer ist. Alternativ wird ungeachtet des tatsächlich Füllstandes des Behälters davon ausgegangen, dass der Behälter bei Unterschreiten des vorbestimmten Füllstandswerts nicht ausreichend befüllt ist.

"Leer" und "befüllt" sind nicht darauf beschränkt zu verstehen, dass sich etwa bei leerem Behälter keinerlei Fluid mehr in diesem befindet bzw. kein weiteres Fluid in den Behälter passen würde.

Ein Erreichen oder Unterschreiten des vorbestimmten Füllstandwerts kann vielmehr als eine Information für die Steuervorrichtung verstanden werden, die auf eine solche Information z. B. mit dem Öffnen oder Schließen eines Ventils zu einem Zulauf reagieren kann.

In manchen beispielhaften, erfindungsgemäßen Ausführungsformen gibt der Füllstandswert an, dass der Behälter vollständig befüllt ist. Alternativ wird ungeachtet des tatsächlich Füllstandes des Behälters davon ausgegangen, dass der Behälter bei Erreichen des vorbestimmten Füllstandswerts ausreichend oder vollständig befüllt ist.

In manchen beispielhaften, erfindungsgemäßen Ausführungsformen wird der Heizvorgang erst dann gestartet, wenn der Füllstand den vorbestimmten Füllstandswert mehrfach, insbesondere zweimal, dreimal, viermal oder fünfmal, erreicht hat.

In manchen beispielhaften, erfindungsgemäßen Ausführungsformen beginnt das Starten des Heizvorgangs zum Erwärmen des Fluids im Heizbehälter nur dann, nachdem ein Zufluss in den Vorlauf und/oder ein Ablauf aus dem Behälter unterbunden wurde oder nicht erfolgte, beispielsweise nach einem Stopp der Dialysevorrichtung oder des Zulaufs, z. B. wegen eines Alarms.

In manchen beispielhaften, erfindungsgemäßen Ausführungsformen beginnt das Starten des Heizvorgangs zum Erwärmen des Fluids im Heizbehälter nur, falls ferner (oder sobald ferner) ein Fluid aus einer Bilanzkammer der Blutbehandlungsvorrichtung herausströmt. Dies gilt insbesondere, nachdem der Fluss aus der Bilanzkammer unterbunden wurde oder nicht erfolgte.

Mit einer Bilanzkammer wird immer ein vorbestimmtes Volumen gepumpt, wobei zwischen Phasen, in welchen die Volumina gepumpt werden, Ventilschaltphasen liegen, in denen die Ventile der Bilanzkammer umgeschaltet werden. Während der Ventilschaltphasen kann kein Fluss vorliegen. Diese Ventilschaltphasen gehören im Sinne dieser Beschreibung dennoch zu den Phasen, in denen ein Fluss vorliegt. Bei einer kontinuierlich arbeitenden Bilanzkammer liegt mit anderen Worten gemäß dieser Beschreibung auch während der Ventilschaltphasen ein Fluss vor, und das Starten der Heizung wird nicht in diesen Ventilschaltphasen gemäß des erfindungsgemäßen Verfahrens gesteuert, wenn es dadurch zu einem kurzzeitigen Stoppen des Flusses kommt. In diesem Sinne sind Phasen, die auf Grund der Konstruktion bzw. Ansteuerung der Pumpvorrichtung oder der Bilanzkammer, mit der ein diskontinuierlicher beziehungsweise pulsatiler Fluss generiert wird, ebenfalls als Flussphasen zu verstehen, also nicht als Phasen, in denen der Fluss aus der Bilanzkammer unterbunden gewesen wäre.

In manchen beispielhaften, erfindungsgemäßen Ausführungsformen ist der Heizbehälter stromauf oder stromab des Behälters angeordnet, beide Behälter stehen mittels einer oder mehrerer Verbindungsleitungen in Fluidverbindung miteinander. Der Behälter und der Heizbehälter können eine Einheit miteinander bilden und unmittelbar miteinander verbunden sein. Der Behälter und der Heizbehälter können in einer Einheit oder in einem Gehäuse angeordnet sein und beispielsweise mittels einer Trennwand und/oder einem Überlaufsystem und/oder einem Schlauchsystem oder einem Rohrsystem miteinander in Fluidverbindung stehen.

In manchen beispielhaften, erfindungsgemäßen Ausführungsformen ist das Starten des Heizvorgangs ein Verstärken der Heizanstrengung, ausgehend von einem niedrigen Heizniveau, bei welchem die Heizvorrichtung auch ohne ausreichenden Füllstand oder Fluid im Heizbehälter keinen Schaden infolge eines zu starken Erwärmens und beispielsweise einer Schädigung des Heizbehälters, in dem das Fluid bei normalem Betrieb fließt und mittels der Heizvorrichtung aufgeheizt wird oder eines "Durchbrennens" eines Heizstabs nimmt. Bei diesen Ausführungsformen kann das Starten der Heizaktivität als ein Verlassen beispielsweise eines "Stand by"-Heizmodus zu verstehen sein.

In manchen beispielhaften, erfindungsgemäßen Ausführungsformen kann es sich bei der Heizvorrichtung um einen Heizstab handeln. Dieser Heizstab kann von unten oder von oben in den Heizbehälter eingeführt sein. Der Heizstab kann derart aufgebaut sein, dass er zwei Bereiche aufweist. Im ersten Bereich ist das das Heizen erzeugende Element, beispielsweise eine Heizspirale, angeordnet, und im zweiten Bereich ist dieses Element nach außen kontaktiert. Der erste Bereich ist dabei an dem der Kontaktierung entfernten Ende des Heizstabs angeordnet. Bei einem von unten eingeführten Heizstab führt ein Absenken des Flüssigkeitspegels unter die Höhe des Heizstabs beziehungsweise das Ausbilden eines Luftpolsters am oberen Ende des Heizstabs unmittelbar dazu, dass ein stark aufgeheizter Bereich des ersten Bereichs des Heizstabes nicht von Flüssigkeit bedeckt ist und damit schnell oder schneller zu einer potentiellen Schädigung durch Überhitzen. Bei einem von oben eingeführten Heizstab wird zunächst der zweite Bereich vom Fluid freigegeben, so dass hier ein Luftpolster im Heizbehälter weniger rasch zum Problem einer Schädigung durch ein Überheizen führen mag.

In manchen beispielhaften, erfindungsgemäßen Ausführungsformen ist das Anhalten des Heizvorgangs ein Verringern der Heizanstrengung, ausgehend von einem höheren Heizniveau, und/oder beispielsweise das Überführen der Heizaktivität in einen Heizmodus, in welchem die Heizvorrichtung auch ohne ausreichenden Füllstand oder Fluid im Heizbehälter keinen Schaden infolge eines zu starken Erwärmens und beispielsweise eines "Durchbrennens" eines Heizstabs oder eines Überhitzens nimmt. Bei diesen Ausführungsformen kann das Anhalten der Heizaktivität als ein Überführen beispielsweise in einen "Stand by"-Heizmodus zu verstehen sein.

In manchen beispielhaften, erfindungsgemäßen Ausführungsformen wird der Heizvorgang erst dann gestartet, wenn der Füllstand zunächst unter den vorbestimmten Füllstandswert gefallen ist und anschließend, d. h. nach einem Befüllen des Behälters, den vorbestimmten Füllstandswert erreicht oder überschreitet. Anders ausgedrückt muss der Füllstand in diesen Ausführungsformen zunächst bis auf den einen Füllstand abgefallen sein, welcher beispielsweise einen leeren oder nicht mehr ausreichend gefüllten Behälter anzeigt. Anschließend, also erst nachdem der Behälter zunächst nicht ausreichend befüllt war, füllt sich der Behälter bis wenigstens zum vorbestimmten Füllstand oder Füllstandswert und erst nach dessen Erreichen wird der Heizvorgang gestartet. Mit diesem Ablauf kann sichergestellt werden, dass der Heizbehälter tatsächlich befüllt sein muss und ein Starten des Heizvorgangs mit einem leeren Heizbehälter ausgeschlossen werden kann. Dies gilt insbesondere dann, wenn der Heizbehälter dem Behälter vorgeschaltet ist. In diesem Fall kann davon ausgegangen werden, dass der Behälter nur dann gefüllt sein kann, wenn der Heizbehälter ebenfalls gefüllt ist. Damit wird sichergestellt, dass der Heizvorgang nur bei ausreichend befülltem Heizbehälter gestartet wird.

In manchen beispielhaften, erfindungsgemäßen Ausführungsformen wird der Füllstand des Behälters mittels eines mechanischen Füllstandsmessers, eines Leitfähigkeitssensors, eines optischen Sensors oder einer beliebigen Kombination hiervon bestimmt.

Ein mechanischer Füllstandsmesser ist beispielsweise ein Schwimmer, der sich mit einem Füllstandspegel mitbewegt. Mit einem an den Schwimmer angeschlossenen oder verbundenen Positionssensor kann der Füllstand als elektrisches Signal in eine Regelvorrichtung oder Steuervorrichtung weitergeführt werden. Der mechanische Füllstandsmesser kann als mechanischer Sensor bezeichnet werden.

Der Füllstandssensor kann einen Schalter aufweisen, beispielsweise einen Schwimmerschalter. Ist der Schalter geöffnet, gilt der Behälter als gefüllt und/oder der vorbestimmte Füllstandswert erreicht. Ist der Schalter geschlossen, so wird der Behälter nachgefüllt. Damit wird sichergestellt, dass eine gewisse Füllmenge nicht unterschritten wird mit hiermit verbundenen Vorteilen, dass die eine erforderliche Reaktionszeit bietet, und damit der Heizbehälter nicht leer läuft. Eine mit dem Schalter verbundene Schaltung kann ausgestaltet sein, um eine gewisse Hysterese zu berücksichtigen, damit bei einem bewegten Wasserfüllstand nicht auch dann nachgefüllt wird, wenn dies noch nicht erforderlich ist.

In manchen beispielhaften, erfindungsgemäßen Ausführungsformen wird das Fluid aus dem Zulauf zunächst in einem Fluidreservoir gespeichert. Anschließend fließt das Fluid in den Heizbehälter und daran anschließend mittels Überlaufs aus dem Heizbehälter in den Behälter.

In manchen beispielhaften, erfindungsgemäßen Ausführungsformen wird das Fluid zusätzlich mittels eines Wärmetauschers erwärmt. Der Wärmetauscher kann Dialysat (also verbrauchte oder aus dem Dialysator abgeführte Dialysierflüssigkeit) aus dem Dialysierflüssigkeitskreislauf führen und Wärme des Dialysats auf das Fluid übertragen. Das Dialysat kann anschließend stromab des Wärmetauschers entsorgt werden.

In manchen beispielhaften, erfindungsgemäßen Ausführungsformen wird Fluid, das mittels der Heizvorrichtung und/oder dem Wärmetauscher erwärmt wurde, mittels einer Bilanzkammer aus dem Vorlauf des Dialysierflüssigkeitskreislaufs in den Dialysierflüssigkeitskreislauf gefördert.

In manchen beispielhaften, erfindungsgemäßen Ausführungsformen weist der Heizbehälter einen Temperatursensor auf, konfiguriert zum Messen der Temperatur des Fluids im Heizbehälter.

In manchen beispielhaften, erfindungsgemäßen Ausführungsformen muss zum Starten des Heizvorgangs der Füllstand des Behälters den vorbestimmten Füllstandswert über einen vorbestimmten Zeitraum T1 hinweg angenommen oder überschritten haben, insbesondere in einem Rezirkulationsmodus.

Das Starten der Heizung nach einer vorbestimmten Zeitdauer kann insbesondere in einem Rezirkulationsmodus aktiviert sein. Die Blutbehandlungsvorrichtung kann in verschiedenen Modi betreibbar sein, beispielsweise einem Behandlungsmodus und einem Rezirkulationsmodus. In dem Behandlungsmodus wird Flüssigkeit dem Hydrauliksystem, den Vorlauf und den Dialysatkreislauf aufweisend, zugeführt und abgeführt, während im Rezirkulationsmodus die Flüssigkeit zirkuliert, ohne dass dem System Flüssigkeit zugeführt wurde oder entnommen wird. Dadurch bleibt der Pegel im Behälter weitestgehend konstant, weshalb der Füllstandsmesser oder Schwimmer einen "gefüllten" Behälter angibt. Dieser Rezirkulationsmodus kann beispielweise zumindest während eines Reinigungsmodus, insbesondere eines Heißreinigens, aktiviert sein, in dem die Heizvorrichtung gestartet werden muss, auch wenn der Füllstandsmesser oder Schwimmer keine Bewegung macht, die einen Füllvorgang des Behälters anzeigen würde. Um bei gleichbleibendem Füllstand ein Starten der Heizung zu ermöglichen, wird in diesem Modus obiges Starten nach Ablauf einer vorbestimmten Zeitdauer, während welcher der vorbestimmte Füllstandswert durchgehend erreicht war, erlaubt.

In manchen beispielhaften, erfindungsgemäßen Ausführungsformen wird wenigstens einer der folgenden Schritte mittels der Steuervorrichtung gesteuert oder geregelt: das Starten oder Beenden des Heizvorgangs im Heizbehälter; das Überwachen des Füllstands im Behälter oder des Füllstandsensors; und das Öffnen oder Schließen eines Absperrventils zum Befüllen des Fluidreservoirs und/oder des Behälters mittels des Zulaufs.

In manchen beispielhaften, erfindungsgemäßen Ausführungsformen umfasst die Blutbehandlungsvorrichtung wenigstens den Behälter, eine Einrichtung zum Erkennen, ob der vorbestimmte Füllstandswert des Behälters erreicht ist, einen mit dem Behälter in Fluidverbindung stehenden Heizbehälter mit einer Heizvorrichtung zum Erwärmen des Fluids und eine Einrichtung zum Weiterleiten des Fluids in den Dialysierflüssigkeitskreislauf der Blutbehandlungsvorrichtung.

In manchen beispielhaften, erfindungsgemäßen Ausführungsformen weist die Einrichtung zum Befüllen oder Weiterleiten des Fluids in den Dialysierflüssigkeitskreislauf eine Bilanzkammer auf oder ist als solche ausgestaltet.

In manchen beispielhaften, erfindungsgemäßen Ausführungsformen weist die Blutbehandlungsvorrichtung ein Reservoir auf, welches angeordnet ist, um mit Fluid, z. B. aus einem Zulauf oder externen Zulauf, befüllt zu werden.

In manchen beispielhaften, erfindungsgemäßen Ausführungsformen weist die Blutbehandlungsvorrichtung einen Wärmetauscher und/oder wenigstens einen Anschluss für eine Konzentratzugabe auf.

In manchen beispielhaften, erfindungsgemäßen Ausführungsformen wird das Fluid in einem ersten Schritt in einem Fluidreservoir gespeichert, bevor es in einem zweiten Schritt aus dem Fluidreservoir aktiv oder passiv in den Behälter zum Bestimmen des Fluid-Füllstands überläuft. In diesen Ausführungsformen ist zwischen Fluidreservoir und dem Behälter kein Heizbehälter angeordnet. Der Heizbehälter kann stromab des Behälters angeordnet oder nachgeschaltet sein.

In manchen beispielhaften, erfindungsgemäßen Ausführungsformen ist stromab des Behälters der Heizbehälter angeordnet.

In manchen beispielhaften, erfindungsgemäßen Ausführungsformen weist der Vorlauf des Dialysierflüssigkeitskreislaufs eine Entgasungskammer zum Entgasen des Fluids und/oder wenigstens eine Luftabscheidekammer auf.

Beispielsweise wird zunächst mittels einer Entgasungsdrossel, die in einer fluidführenden Leitung angeordnet ist, ein Unterdruck im Fluid erzeugt und hierdurch Gas (meist Luft) aus dem Fluid freigesetzt. In der stromab nachfolgenden Entgasungskammer wird das freigesetzte Gas gesammelt. In einer weiter stromab nachfolgenden Luftabscheidekammer kann das Gas mittels einer abführenden Leitung abgeführt werden. In der Luftabscheidekammer kann weiterhin Luft aus anderen Quellen, z. B. aus zuführenden Konzentratleitungen aufgefangen, gesammelt und abgeschieden werden. Ein Entgasen und/oder ein Luftabscheiden kann vorteilhaft ein Zuführen von Luft in den Dialysekreislauf verhindern.

Die erfindungsgemäße Blutbehandlungsvorrichtung oder Blutbehandlungsmaschine kann zur Dialyse, Hämodialyse, Hämodiafiltration, Filtration oder Apherese verwendbar sein.

In manchen beispielhaften, erfindungsgemäßen Ausführungsformen ist die Steuervorrichtung zur Steuerung und/oder Regelung einzelner Parameter programmiert. Die Parameterzustände können beispielsweise das Einschalten und Abschalten der Heizvorrichtung betreffen. Die Steuervorrichtung kann einen elektrischen Schaltkreis mit einem Netzteil zur Spannungsversorgung umfassen. Die Steuervorrichtung kann als Steuergerät bezeichnet werden. Zur Ansteuerung der Heizvorrichtung können sogenannte Triacs (engl.: Triode for Alternating Current) verwendet werden. Mittels der Steuervorrichtung kann somit das Anschalten oder Abschalten des Heizvorgangs im Heizbehälter gesteuert und/oder geregelt werden. Weiterhin können mittels der Steuervorrichtung der Füllstandssensor überwacht und/oder ein Absperrventil zum Befüllen des Fluidreservoirs geöffnet oder geschlossen werden.

In einigen beispielhaften, erfindungsgemäßen Ausführungsformen weist die Messvorrichtung, der mechanische Füllstandsmesser und/oder der mechanische Sensor nur zwei unterschiedliche, sich gegeneinander ausschließende, binäre Zustände auf, z. B. Füllstandswert H1 erreicht oder nicht, Behälter voll oder nicht, Behälter leer oder nicht. Die Messvorrichtung, der mechanische Füllstandsmesser und/oder der mechanische Sensor geben dabei in einigen Ausführungsformen ein Steuersignal an die Steuervorrichtung, das nur zwischen zwei Zuständen unterscheidet. Dies umfasst beispielsweise, dass die Messvorrichtung, der mechanische Füllstandsmesser und/oder der mechanische Sensor in einigen Ausführungsformen eine Hysterese aufweisen, d. h. dass der Zustand von einem vorherigen Zustand abhängig ist; z. B. kann der Zustand "Behälter voll" einen anderen Schwellwert haben, wenn der Schwellwert von oben kommend erreicht wird, als wenn er von unten kommend erreicht wird. In einigen beispielhaften, erfindungsgemäßen Ausführungsformen wird die Hysterese ganz oder zumindest teilweise durch die Steuervorrichtung erzeugt.

In einigen Ausführungsformen ist die Messvorrichtung keine Vorrichtung, die mehr als zwei Zustände annehmen kann, insbesondere ist die Messvorrichtung in solchen Ausführungsformen kein Hall-Effekt-Positionsgeber.

In einigen beispielhaften, erfindungsgemäßen Ausführungsformen weist die Steuervorrichtung keinen PID-Regler (proportional-integral-derivative controller) auf, insbesondere keinen für die Wasserzufuhr.

In einigen beispielhaften, erfindungsgemäßen Ausführungsformen ist ein Regler der Steuervorrichtung, insbesondere für die Wasserzufuhr, kein stetiger Regler, insbesondere ein unstetiger Regler, insbesondere kein Zweipunktregler.

In einigen beispielhaften, erfindungsgemäßen Ausführungsformen ist der Behälter und/oder der Heizbehälter keine Entgasungskammer und/oder Luftabscheidekammer.

In einigen beispielhaften, erfindungsgemäßen Ausführungsformen ist dem Behälter und/oder dem Heizbehälter eine Entgasungskammer und/oder Luftabscheidekammer vor- und/oder nachgeschaltet.

In einigen beispielhaften, erfindungsgemäßen Ausführungsformen sind die Messeinrichtung, der mechanischer Füllstandsmesser, der mechanische Sensor und/oder der Schwimmer im Behälter angeordnet und insbesondere nicht in einer vor- oder nachgeschalteten Entgasungskammer und/oder Luftabscheidekammer.

In einigen beispielhaften, erfindungsgemäßen Ausführungsformen enthält der Behälter und/oder der Heizbehälter keine Körper, insbesondere keine Kunststoffkörper, zur Entgasung der im Behälter enthaltenen Flüssigkeit. Insbesondere enthält in einigen beispielhaften, erfindungsgemäßen Ausführungsformen der Behälter und/oder Heizbehälter keine Polypropylenkörper wie z.B. Zylinder und/oder Kugeln.

In einigen beispielhaften, erfindungsgemäßen Ausführungsformen schaltet die Steuervorrichtung ein Ventil im Zulauf nicht mit, oder während, einer festen Periodendauer an und/oder aus.

In einigen beispielhaften, erfindungsgemäßen Ausführungsformen berechnet die Steuervorrichtung kein Schaltverhältnis und/oder keinen Tastgrad des Ventils im Zulauf.

In einigen beispielhaften, erfindungsgemäßen Ausführungsformen befindet sich im Zulauf kein Sensor, der das Einströmen des Fluids aus dem Zulauf in den Behälter oder in das Fluidreservoir misst. In anderen Ausführungsformen ist mindestens ein solcher Sensor vorhanden.

In einigen beispielhaften, erfindungsgemäßen Ausführungsformen steuert die Steuervorrichtung den Zustand der Heizung nicht in Abhängigkeit von einem gemessenen Fluss im Zulauf.

Einige oder alle erfindungsgemäßen Ausführungsformen können einen oder mehrere der oben oder im Folgenden genannten Vorteile aufweisen.

Das erfindungsgemäße Verfahren kann eine Schädigung der Heizvorrichtung und/oder des Heizbehälters, welche zum Erwärmen eines Fluids in einem Vorlauf zu einem Dialysierflüssigkeitskreislauf vorgesehen sind, vorteilhaft verhindern.

Das erfindungsgemäße Verfahren kann ein Überhitzen der Heizvorrichtung aufgrund einer beispielsweise zu früh oder zu einem anderen nicht gewünschten Zeitpunkt einsetzenden Erwärmung vorteilhaft verhindern. Dies kann beispielsweise der Fall sein, nachdem Fluid aus dem Vorlauf in den Dialysierflüssigkeitskreislauf gefördert wurde und noch keine ausreichende Menge frischen Fluids z. B. aus dem Zulauf in den Heizbehälter nachfließen konnte, ein Zustand, der als Leerlaufen bezeichnet werden kann, jedenfalls aber zu einem ungenügend gefüllten Heizbehälter führen kann. Ein Betreiben der Heizvorrichtung, ohne dass der Heizbehälter zumindest ausreichend gefüllt ist, kann die Heizvorrichtung schädigen. Dies gilt es aus Sicherheitsgründen, aus ökonomischen Gründen und/oder aus medizinischen Gründen zu vermeiden. Die vorliegende Erfindung kann dazu beitragen, eine solche Schädigung zu verhindern.

Ferner kann das Starten der Heizvorrichtung, solange noch kein Fluss existiert, zu einem starken lokalen Aufheizen der Flüssigkeit führen, was zu einer Schädigung des Heizbehälters oder der mit diesem verbundenen Schläuche führen kann. Im Extremfall kann das starke Aufheizen zu einem Verdampfen der Flüssigkeit führen. Auch ein Aufheizen eines leeren Heizbehälters kann zu einer Schädigung führen, wobei ein Sicherstellen eines gefüllten Heizbehälters mittels des hierin beschriebenen erfindungsgemäßen Verfahrens vorteilhaft möglich ist.

Zudem kann erfindungsgemäß das geförderte Volumen so wählbar sein, dass der Heizbehältervorteilhaft gefüllt sein muss.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- **Fig. 1**: zeigt eine Vorrichtung einer ersten erfindungsgemäßen Ausführungsform der Blutbehandlungsvorrichtung mit einem Behälter zur Füllstandsmessung und einem stromauf hierzu angeordneten Heizbehälter mit einer Heizvorrichtung;
- **Fig. 2**: zeigt eine Vorrichtung einer zweiten erfindungsgemäßen Ausführungsform der Blutbehandlungsvorrichtung mit einem Behälter zur Füllstandsmessung und einem stromab hierzu angeordneten Heizbehälter mit einer Heizvorrichtung;
- **Fig. 3**: zeigt schematisch vereinfacht eine beispielhafte Ausführungsform des erfindungsgemäßen Verfahrens mit einem Erkennen einer Füllstandsänderung und einem Starten des Heizvorgangs; und
- **Fig. 4**: zeigt schematisch vereinfacht eine beispielhafte Ausführungsform des erfindungsgemäßen Verfahrens zum Regeln und/oder Überwachen einer Heizvorrichtung zum Erwärmen eines Fluids.

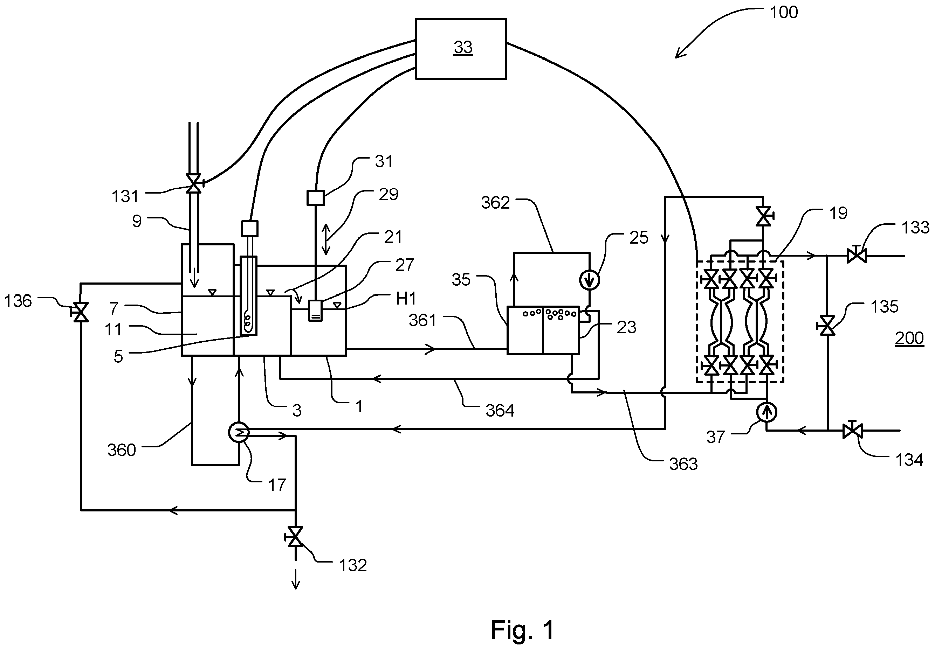

**Fig. 1** zeigt eine Vorrichtung 100 einer ersten, rein exemplarischen Ausführungsform mit einem Behälter 1 für ein Fluid 11. Die Vorrichtung 100 ist mit einer erfindungsgemäßen Blutbehandlungsvorrichtung 200 verbunden oder Teil hiervon. Der Behälter 1 dient zumindest der Füllstandsmessung des Fluids 11. Der Füllstand bezieht sich auf den Behälter 1. Gemessen wird somit im Behälter 1. Die Vorrichtung 100 weist ferner einen stromauf (in Fig. 1 links) des Behälters 1 angeordneten Heizbehälter 3 mit einer Heizvorrichtung 5 zum Wärmen oder Heizen des Fluids 11, wenn dieses im Heizbehälter 3 vorliegt, auf. Der Behälter 1 und der Heizbehälter 3 sind in dieser Ausführungsform exemplarisch als eine Einheit (als physische Einheit) ausgeführt.

Stromauf des Heizbehälters 3 kann ferner ein Reservoir 7 angeordnet sein, in welches das Fluid 11 aus einem Zulauf 9 einfließen kann. Der Zulauf 9 kann als externer Zulauf 9 bezeichnet werden, da das Fluid 11 hier exemplarisch aus einem externen Leitungsabschnitt, beispielsweise aus einem Wasserversorgungssystem, zugeführt wird. Der Zulauf 9 kann mittels eines Absperrventils 131 geöffnet und geschlossen werden.

In der Ausgestaltung der Fig. 1 steht der Zulauf 9 mit dem Wasserversorgungssystem in Verbindung. Das Fluid 11 ist somit Wasser und wird im Folgenden auch als Wasser 11 bezeichnet. Das Fluid soll hierdurch jedoch nicht auf Wasser beschränkt werden.

Das Wasser 11 kann optional durch eine Verbindungsleitung 360, die zwischen dem Heizbehälter 3 und dem Reservoir 7 angeordnet sein kann, aus dem Reservoir 7 in den Heizbehälter 3 fließen. Ebenso kann eine direkte Verbindung zwischen dem Heizbehälter 3 und dem Reservoir 7, beispielsweise mittels einer teilweise durchlässigen Trennwand zwischen beiden Behältern, bestehen. Die Verbindungsleitung 360 ist optional mit einem Wärmetauscher 17 verbunden. Die Fließrichtung des Wassers 11 ist mittels Pfeilen dargestellt. Die Wasserpegel sind im Heizbehälter 3 und im Reservoir 7 optional gleich, das heißt, es findet mittels der Verbindungsleitung 360 ein Druckausgleich zwischen beiden Behältern statt.

Der Wärmetauscher 17 kann das Wasser 11 zusätzlich zur Heizvorrichtung 5 erwärmen. Das den Wärmetauscher 17 durchströmende, wärmere Fluid wird dem Wärmetauscher 17 aus einer Bilanzkammer 19 zugeführt und ist insbesondere verbrauchtes Dialysat aus einem der Bilanzkammer 19 nachgeschalteten, in Fig. 1 nicht gezeigten Dialysierflüssigkeitskreislauf der erfindungsgemäßen Blutbehandlungsvorrichtung 200. Das Dialysat fließt nach Durchströmen des Wärmetauschers 17 beispielsweise in ein Entsorgungssystem ab. In der aus dem Wärmetauscher 17 abführenden Leitung ist rein exemplarisch ein weiteres Absperrventil 132 angeordnet. "Verbrauchtes Dialysat" bezeichnet hierbei Dialysat, das wenigstens einen oder mehrere Abschnitte des Dialysierflüssigkeitskreislaufs durchströmt, die während einer Behandlung stromab des zur Blutbehandlung verwendeten Dialysators angeordnet sind. Der Dialysator ist dabei stromab des Ventils 133 und stromauf des Ventils 134 angeordnet.

Das im Heizbehälter 3 mittels der Heizvorrichtung 5 weiter erwärmte Wasser 11 fließt mittels eines Überlaufs, der durch die Überlaufrichtung 21 angedeutet ist, in den Behälter 1.

Der Wasserpegel oder Füllstand des Behälters 1 wird mittels eines Sensors oder Füllstandsmessers bestimmt, der in Fig. 1 exemplarisch als ein mechanischer Füllstandsmesser 27, der als mechanischer Sensor oder Schwimmer 27 bezeichnet werden kann, ausgeführt ist. Der Füllstandsmesser 27 kann alternativ oder ergänzend beispielsweise ein Leitfähigkeitssensor, ein optischer Sensor oder ein anderer Sensor sein.

Die Lage oder Position des Schwimmers 27 verändert sich je nach Füllstand oder Pegel in dem Behälter 1 in einer vertikalen Verschieberichtung 29 des Schwimmers 27. Eine schematisch dargestellte Messeinrichtung 31 detektiert einen, insbesondere vorbestimmten, Füllstandswert H1, der einen gefüllten, oder zumindest weitgehend gefüllten Behälter 1 anzeigt oder signalisiert. Alternativ könnte der Schwimmer 27 in einer anderen, hier nicht dargestellten Ausführungsform die jeweils aktuelle Position des Schwimmers 27 mittels der schematisch dargestellten Messeinrichtung 31 messen oder signalisieren.

Das Messsignal der Messeinrichtung 31 wird an eine Steuer- oder Regelvorrichtung 33 (kurz: Steuervorrichtung 33) weitergeleitet. In der Steuervorrichtung 33 können verschiedene Messsignale aufgenommen, verarbeitet und ausgegeben werden.

Das erwärmte Wasser 11 fließt von dem Behälter 1 über eine Verbindungsleitung 361 in eine Entgasungskammer 35. Optional kann in der Verbindungsleitung 361 eine sogenannte Entgasungsdrossel angeordnet sein, die mittels Unterdruck Gas aus dem Wasser freisetzen kann. In der Entgasungskammer 35 wird aus dem Wasser 11 gelöstes Gas gesammelt, welches in der Entgasungskammer 35 nach oben steigt (dies wird durch kleine Luftblasen im oberen Bereich der Entgasungskammer 35 angedeutet).

Anschließend fließt das Wasser 11 über eine weitere Verbindungsleitung 362 in einen Luftabscheider 23. In der Verbindungsleitung 362 ist eine Pumpe 25 angeordnet. Mittels dieser Pumpe 25 wird das Wasser 11 wenigstens aus dem Behälter 1 in die Entgasungskammer 35, von dort in den Luftabscheider 23 und von dort über eine weitere Verbindungsleitung 363 weiter in die Bilanzkammer 19 gepumpt oder gefördert. Optional kann zumindest ein Teil des Wassers 11 aus dem Luftabscheider 23 über die Verbindungsleitung 364 zurück in den Heizbehälter 3 geführt werden. Beispielsweise kann ein besonders schaumdurchsetztes Wasser 11 zurückgeführt werden, welches nicht über die Verbindungsleitung 363 in die Bilanzkammer 19 gelangen soll.

Zu den Steuer- oder Regelsignalen (kurz: Steuersignale), die von der Steuervorrichtung 33 ausgegeben werden, ist wenigstens das Steuersignal zum Anschalten und Abschalten der Heizvorrichtung 5 zu zählen. Weiterhin können das Absperrventil 131 zum Öffnen und Schließen des Zulaufs 9 sowie die Bilanzkammer 19, beispielsweise zum Öffnen und Schließen einzelner oder mehrerer Ventile, angesteuert werden.

Erfindungsgemäß wird der Heizvorgang zum Erwärmen oder Heizen des Wassers 11 im Heizbehälter 3 durch Anschalten der Heizvorrichtung 5 nur dann gestartet, wenn der Füllstand in dem Behälter 1 einen vorbestimmten Füllstandswert H1 erreicht hat.

In einer exemplarischen Ausführungsform des erfindungsgemäßen Verfahrens wird die Heizvorrichtung 5 wie nachfolgend beschrieben gestartet - etwa durch Einschalten oder Zuschalten von sogenannten Heizungstrioden für Wechselstrom im Netzteil durch einen Prozessor in der Steuervorrichtung 33.

Der Schwimmer 27 kann in dem Behälter 1 zunächst in einer Ausgangsposition eine beliebige Position annehmen, die ober- oder unterhalb des vorbestimmten Füllstandwerts H1 liegt, beispielsweise im unteren Bereich des Behälters 1 (dies würde einen leeren Behälter 1 signalisieren), eine mittlere Position oder eine andere Position. Erreicht der Schwimmer 27 zumindest den vorbestimmten Füllstandswert H1, so bedeutet das einen erfolgten Zulauf in den Behälter 1. Dieser Zulauf erfolgt insbesondere mittelbar über den Zulauf 9 bei einem geöffneten Ventil 131. Der Zulauf in den Behälter 1 erfolgt nicht unmittelbar aus dem Zulauf 9, sondern mittelbar über das Reservoir 7, den Heizbehälter 3 und den Überlauf 21. Der vorbestimmte Füllstandswert H1 kann eine obere Begrenzung des Schwimmers 27 darstellen.

Bei Erreichen des vorbestimmten Füllstandswert H1 wird erfindungsgemäß der Heizvorgang zum Erwärmen des Wassers 11 im Heizbehälter 3 gestartet. Optional sollten vor einem Anschalten der Heizvorrichtung 5 keine anderen Signale aktiv sein, wie beispielsweise ein Flussalarm oder ein Wasseralarm, die einen Mangel an Wasser 11 an einer anderen Stelle, beispielsweise im Reservoir 7 oder in der Verbindungsleitung, anzeigen könnten.

In einer exemplarischen, erfindungsgemäßen Ausführungsform, im sogenannten Rezirkulationsmodus, ist der Vorlauf vollständig mit Wasser 11 (oder einem anderen Fluid) gefüllt, das Absperrventil 131 dauerhaft geschlossen und die Verbindung zur Blutbehandlungsvorrichtung 200 kurzgeschlossen. Das Kurzschließen zur Blutbehandlungsvorrichtung 200 erfolgt exemplarisch mittels Schließens der Ventile 133 und 134 und Öffnen des Ventils 135.

Alternativ liegt der Kurzschluss während des Reinigungsmodus oder Rezirkulationsmodus hinter den beiden Ventilen133 und 134, also z. B. stromab des Ventils 133 und stromauf des Ventils 134 (anders als hier gezeigt). Optional bedarf es keines Ventils 135. In dieser Ausführungsform kann der Kurzschluss dadurch hergestellt werden, dass die während der Behandlung mit dem Dialysator verbundenen Enden des Dialysierkreislaufs direkt oder indirekt miteinander verbunden sind oder werden.

Weiterhin wird die aus dem Wärmetauscher 17 abführende Leitung mittels des Absperrventils 132 geschlossen. Das aus dem Wärmetauscher 17 abfließende Wasser 11 wird durch Öffnen des Ventils 136 in das Reservoir 7 zurückgeführt. Mittels dieser Anordnung wird ein geschlossener Kreislauf im Vorlauf hergestellt. Somit zeigt der Schwimmer 27 dauerhaft den vorbestimmten Füllstandswert H1 an. Ein Erreichen oder Überschreiten des vorbestimmten Füllstandswerts H1 mittels Zulauf ist somit im Rezirkulationsmodus nicht möglich. Trotz Fehlen des Merkmals "Erreichen des Füllstandswert H1" soll die Heizungsvorrichtung 5 dauerhaft angeschaltet sein, um beispielsweise ein Reinigen des Vorlaufs mit einem erwärmten Wasser 11 durchführen zu können. Um dieses dauerhafte Heizen im Rezirkulationsmodus mittels der Steuervorrichtung 33 sicherzustellen, können verschiedene Schaltmodi in der Steuervorrichtung 33 vorgesehen sein. Ein Schaltmodus kann beispielsweise vorsehen, dass über eine vorgesehene Zeit eine Variable abgefragt wird, die ein Detektieren eines Erreichens oder Überschreitens des vorbestimmten Füllstandswerts H1 des Schwimmers 27 erkennt. Die vorgesehene Zeit kann rein exemplarisch 10 Sekunden, 12,5 Sekunden oder 15 Sekunden betragen. Da im Rezirkulationsmodus kein Füllstandswert H1 erreicht wird, kann dieser Zustand den Rezirkulationsmodus anzeigen oder signalisieren und somit den Heizvorgang anschalten oder dauerhaft angeschaltet lassen. Während des Rezirkulationsmodus ist der Heizbehälter 3 ebenfalls dauerhaft gefüllt oder wird dauerhaft durchströmt. Im Rezirkulationsmodus wird der Fluss oder die Durchströmung mittels der Pumpe 25 erreicht.

Die Bilanzkammer 19 wird mittels eines durch die Pumpe 25 aufzubringenden Drucks und bei entsprechend geöffneten Ventilen über die Verbindungsleitung 363 befüllt. Wenn die Kammern mit Wasser 11 (welches als frisches Dialysat bezeichnet werden kann) aus dem Vorlauf befüllt sind, werden die Ventile, die zum Befüllen offen waren, geschlossen. Wenn anschließend oder zu einem späteren Zeitpunkt verbrauchtes Dialysat aus dem Dialysatkreislauf der angeschlossenen, erfindungsgemäßen Blutbehandlungsvorrichtung 200 abfließen soll, fördert die Pumpe 37 dieses abzubefördernde Dialysat in den vom Wasser 11 abgetrennten Bereich der Bilanzkammer 19. Mittels dieses Förderns von Dialysat in die Bilanzkammer 19 wird gleichzeitig das frische Dialysat aus der Bilanzkammer 19 in den Dialysatkreislauf gefördert. Dies wird dadurch erreicht, dass die Kammern mit dem frischen und verbrauchten Dialysat durch eine Membran getrennt sind und bei einer aktiven Druckausübung auf eine Kammerseite die andere Kammerseite, bei entsprechenden Ventilstellungen, entleert wird.

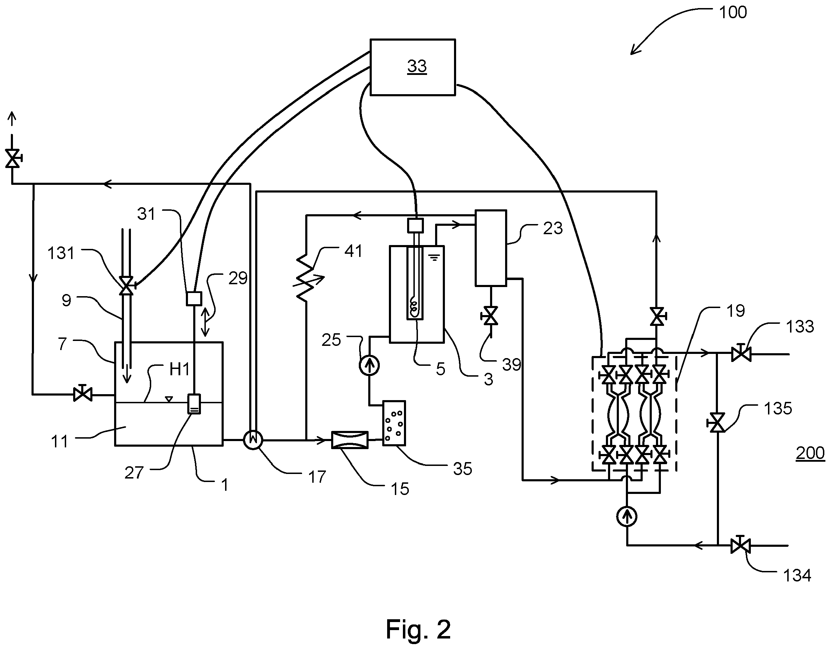

**Fig. 2** zeigt eine Vorrichtung 100 einer zweiten Ausführungsform mit einem Behälter 1 zur Füllstandsmessung und einem stromab von diesem angeordneten Heizbehälter 3 mit einer Heizvorrichtung 5. Die Vorrichtung 100 ist mit einer erfindungsgemäßen Blutbehandlungsvorrichtung 200 verbunden. Der Behälter 1 und der Heizbehälter 3 sind - anders als in Fig. 1 - in dieser Ausführungsform nicht in einer physischen Einheit ausgeführt, sondern räumlich getrennt voneinander.

Wasser 11 wird über den Zulauf 9 und ein ansteuerbares Ventil 131 in den Behälter 1 eingeleitet. Dort kann der Füllstand (oder Pegel) mittels des Schwimmers 27 und der Messeinrichtung 31 bestimmt werden. Der Schwimmer 27 hebt oder senkt sich in der vertikalen Verschieberichtung 29 je nach Füllstand. Von dem Behälter 1 fließt Wasser 11 durch eine Verbindungsleitung mit einem zwischengeschalteten Wärmetauscher 17, in dem das Wasser 11 zusätzlich zu der Heizvorrichtung 5 erwärmt werden kann, und einer Entgasungsdrossel 15 in die Entgasungskammer 35. Stromab der Entgasungskammer 35 sind zunächst eine Pumpe 25 und nachfolgend der mit Wasser 11 befüllte Heizbehälter 3 mit der Heizvorrichtung 5 angeordnet. Weiter stromab ist ein Luftabscheider 23 angeordnet. Über einen Anschluss 39 können Fluide, wie beispielsweise Konzentratlösungen, dem Wasser 11 in dem Luftabscheider 23 für dessen Verwendung als Dialysierflüssigkeit zugegeben werden. Im unteren Bereich des Luftabscheiders 23 führt eine Verbindungsleitung zur Bilanzkammer 19 ab. Durch diese Leitung kann Wasser 11 als frische Dialysierflüssigkeit über die Bilanzkammer 19 einem Dialysierflüssigkeitskreislauf zugeführt werden. Im oberen Bereich des Luftabscheiders 23 führt eine Verbindungsleitung zunächst zu einem optionalen Druckbegrenzungsventil 41 und mündet anschließend in die Verbindungsleitung zwischen dem Behälter 1 und der Entgasungskammer 35.

Mittels der Steuervorrichtung 33 kann das Messsignal der Messeinrichtung 31, welches den vorbestimmten Füllstandswert H1 des Schwimmers 27 im Behälter 1 angibt, erfasst werden. Die Steuervorrichtung 33 kann ein Steuersignal zum Anschalten und Abschalten der Heizvorrichtung 5 ausgeben, das Absperrventil 131 zum Öffnen und Schließen steuern, sowie die Bilanzkammer 19 zum Zuführen von Fluid 11 aus dem Luftabscheider 23 ansteuern.

In einem exemplarischen, erfindungsgemäßen Ausführungsbeispiel wird die Heizvorrichtung 5 durch ein Anschalten eines Heizungsrelais in der Steuervorrichtung 33 gestartet. Das Heizungsrelais wird in diesem Ausführungsbeispiel jedoch nur dann aktiviert, wenn der vorbestimmte Füllstandswert H1 im Behälter 1 mehrmals erreicht wurde, nachdem der Schwimmer zwischenzeitlich jeweils unter den vorbestimmten Füllstandswert H1 abgefallen ist. Beispielsweise kann vorgesehen sein, dass das Heizungsrelais erst dann durch die Steuervorrichtung 33 gestartet werden kann, wenn der Füllstand den vorbestimmten Füllstandswert H1 zunächst unterschreitet, dann beispielsweise viermal überschreitet und zwischenzeitlich jeweils unterschreitet. Alternativ kann das Heizungsrelais nach Durchlaufen der Reihenfolge mit einem erstmaligen Überschreiten beginnen, anschließend unterschreiten und danach viermal, mit einem zwischenzeitlichen Unterschreiten, jeweils wieder überschreitet und den vorbestimmten Füllstandswert H1 wiederholt erreicht.

Zwischen dem Schwimmer 27 bzw. der Messeinrichtung 31 zum Detektieren der aktuellen Schwimmerposition einerseits und der Steuerung des Absperrventils 13 zum Öffnen und Schließen des Zulaufs 9 andererseits gibt es bei der vorliegenden, exemplarischen Ausgestaltung einen direkten Zusammenhang. Das Absperrventil 13 des Zulaufs 9 wird mittels der aktuellen Position des Schwimmers 27 bzw. dem Erreichen des vorbestimmten Füllstandswerts H1 gesteuert. Bei einem fehlerfreien Betrieb wird das Absperrventil 13 geöffnet, wenn der Schwimmer 27 niedrig steht. Strömt Wasser 11 zu, so steigt der Schwimmer 27. Wenn jedoch das Absperrventil 13 des Zulaufs 9 länger als eine vorbestimmte Zeitdauer, z. B. länger als 10 Sekunden, 11 Sekunden oder 12 Sekunden, geöffnet ist und der Schwimmer 27 trotzdem nicht aufsteigt, kann dies auf ein Problem bei der Wasserversorgung durch den Zulauf 9 hindeuten. Beispielsweise kann in einem solchen Fall die Wasserversorgung grundsätzlich unterbrochen sein, wie dies bei einem Rohrbruch, einer Leitungsverstopfung oder einem anderen Problem denkbar ist. Das Erkennen einer unterbrochenen Wasserversorgung ist insbesondere hinsichtlich eines leeren Heizbehälters 3 und einer angeschalteten Heizvorrichtung 5 problematisch und kann zu Schäden an der Heizvorvorrichtung 5 führen.

Mittels der Steuervorrichtung 33 wird zunächst, beispielsweise durch eine elektronische Schaltung, geprüft, ob der Schwimmer 27 den vorbestimmten Füllstandswert H1 aufweist, der einen gefüllten Behälter 1 anzeigt. Fällt diese Prüfung negativ aus, das heißt, der vorbestimmte Füllstandswert H1 ist nicht erreicht, so wird die Bilanzkammer 19 nicht aktiviert und es kann kein Wasser 11 als Dialysierflüssigkeit in den der Bilanzkammer 19 nachgeschalteten Dialysierkreislauf zugeführt werden. Wenn dieser Zustand länger anhält, beispielsweise 10 Sekunden, 11 Sekunden oder 12 Sekunden, kann ein Wassermangelalarm ausgelöst werden. Fällt die Prüfung hingegen positiv aus, das heißt, der vorbestimmte Füllstandswert H1 ist erreicht, so wird die Bilanzkammer 19 aktiviert und es wird optional nach jedem Füllvorgang der Bilanzkammer 19 dieses Wasservolumen beispielsweise aus dem Luftabscheider 23 abgezogen. Der Füllstand des Schwimmers 27 fällt daraufhin unter den vorbestimmten Füllstandswert H1 ab und es wird Wasser aus dem Zulauf 9 durch Öffnen des Absperrventils 13 nachgefüllt. Wenn der Füllstand den vorbestimmten Füllstandswert H1 nicht innerhalb der angegebenen Zeitspanne, z.B. 11 s, wieder erreicht, wird ein Wassermangelalarm aktiviert. Der Wassermangelalarm wird automatisch deaktiviert, sobald Wasser wieder einfließt und der vorbestimmte Füllstandswert H1 erneut erreicht wird.

Neben dem beschriebenen Wassermangelalarm kann gleichfalls ein Wasserüberschussalarm angezeigt werden. Ein Wasserüberschussalarm tritt immer dann auf, wenn der vorbestimmte Füllstandswert H1 dauerhaft über eine längere Zeit angezeigt wird. Dafür können verschiedene Gründe verantwortlich sein, die im Folgenden beschrieben werden. Normalerweise fällt der Füllstand des Schwimmers 27 nach jedem Füllvorgang der Bilanzkammer 19, durch den ein bestimmtes Pumpvolumen aus dem Vorlauf in den Dialysierflüssigkeitskreislauf gefördert wird. Wenn jedoch der Füllstand des Schwimmers 27 auch nach einem zweiten Füllvorgang der Bilanzkammer 19 nicht abfällt, liegt möglicherweise ein Gerätedefekt vor, und es wird optional ein Wasserüberschussalarm ausgegeben oder angezeigt. Das Absperrventil 13 des Zulaufs 9 wird in diesem Fall durch die Steuervorrichtung 33 optional geschlossen. Der Wasserüberschussalarm wird automatisch deaktiviert, sobald der Füllstand des Schwimmers 27 wieder abfällt. Der Wasserüberschussalarm kann weiterhin durch einen Anwender durch Drücken eines sogenannten "Dialysestartschalters" bestätigt werden, wodurch weitere zwei Pumpvorgänge der Bilanzkammer 19 ermöglicht werden, durch die weiteres Wasser entzogen bzw. dem Dialysierflüssigkeitskreislauf zugeführt wird. Die Heizvorrichtung 5 ist in diesem Stadium durch die Steuervorrichtung 33 bereits deaktiviert oder ausgeschaltet.

Die Überwachung des Füllstands des Schwimmers 27 erfolgt wie beschrieben beispielsweise auf Basis eines Zeitraums von ca. 12 Sekunden, um einen Alarm (Wassermangelalarm oder Wasserüberschussalarm) auszulösen. Dies gilt jedoch nicht für den sogenannten Rezirkulationsmodus oder Reinigungsmodus, beispielsweise zum sogenannten Heißspülen. In diesem Modus muss die Heizvorrichtung 5 ständig angeschaltet bleiben. Die Steuervorrichtung 33 überwacht den Rezirkulationsmodus und schaltet den Schwimmer 27 bzw. die Messeinrichtung 31 dauerhaft auf "gefüllt", "oben" oder auf den vorbestimmten Füllstandswert H1, ohne dass ein Zulauf aus dem Zulauf 9 erfolgt. Eine separate Schaltung, die beispielsweise ebenfalls in der Steuervorrichtung 33 angeordnet sein kann, aktiviert und deaktiviert den Rezirkulationsmodus.

Zur näheren Erläuterung des Rezirkulationsmodus wird auf die Beschreibung zur Fig. 1 verwiesen.

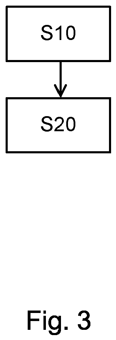

**Fig. 3** zeigt schematisch vereinfacht eine beispielhafte Ausführungsform des erfindungsgemäßen Verfahrens mit einem Erkennen einer Füllstandsänderung im Behälter 1 (S10) und einem Starten des Heizvorgangs (S20). Eine Füllstandsänderung ist insbesondere ein Erreichen (und Anzeigen) des vorbestimmten Füllstandswerts H1. Ein Erreichen des vorbestimmten Füllstandswerts H1 ist als ein "wenigstens einmaliges" Erreichen zu verstehen. Ein mehrmaliges Erreichen bedingt ein zwischenzeitliches Abfallen des Füllstands unter den vorbestimmten Füllstandswert H1. Daher kann das Starten des Heizvorgangs in bestimmten Ausführungsformen erst nach einem mehrmaligen Erreichen des vorbestimmten Füllstandswerts H1 ausgelöst werden.

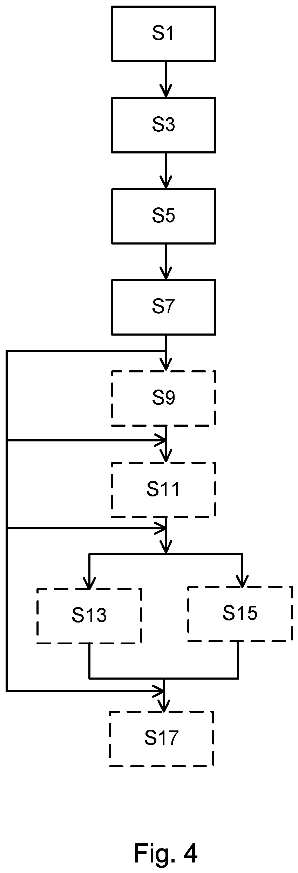

**Fig. 4** zeigt eine schematische Darstellung des erfindungsgemäßen Verfahrens einer beispielhaften Ausführungsform unter Verwendung einer Vorrichtung 100, die mit einer erfindungsgemäßen Blutbehandlungsvorrichtung 200 verbunden oder Teil hiervon ist.

Das Verfahren umfasst in einem optionalen Schritt S1 das Erkennen eines vorbestimmten Füllstandswerts H1 im Behälter 1 der Vorrichtung 100. Das Fluid 11 kann Wasser sein, ohne jedoch auf Wasser beschränkt zu sein. Das Fluid 11 kann eine Mischung von Flüssigkeiten sein.

Das Verfahren umfasst in einem Schritt S3 das Starten der Befüllung des Behälters 1, wenn der Füllstand unterhalb des vorbestimmten Füllstandswerts H1 liegt. Das Befüllen des Behälters 1 kann beispielsweise das Öffnen eines Absperrventils 131 des Zulaufs 9 umfassen. Der Vorgang des Befüllens und des Öffnens des Ventils 131 bedingt, dass zuvor ein Befüllen der Bilanzkammer 19 gestoppt bzw. beendet wurde. Während eines vorherigen Befüllens der Bilanzkammer 19 wiederum muss der Zulauf 9 bzw. das Ventil 131 geschlossen gewesen sein.

Das Verfahren umfasst in Schritt S5 das Erwärmen des Fluids 11 im Heizbehälter 3, oder dessen Beginn, wenn der Füllstand des Behälters 1 den vorbestimmten Füllstandswert H1 erreicht.

Das Verfahren umfasst in Schritt S7 ein optionales Überwachen des Füllstands des Behälters 1. Unterschreitet der Füllstand den vorbestimmten Füllstandswert H1, beispielsweise durch eine Entnahme von Wasser zum Befüllen des Dialysierflüssigkeitskreislaufs, wird der Heizvorgang angehalten bzw. unterbrochen. Der Heizvorgang kann auch vollständig gestoppt werden, also final beendet werden.

In einer weiteren erfindungsgemäßen Ausführungsform kann das Verfahren den optionalen Schritt S9 umfassen, in welchem der Heizvorgang erst dann gestartet wird, wenn der Füllstand zunächst den vorbestimmten Füllstandswert H1 unterschreitet und anschließend, nach einem Befüllen des Behälters 1, den vorbestimmten Füllstandswert H1 erreicht.

Das Verfahren kann in einer erfindungsgemäßen Ausführungsform den optionalen Schritt S11 beinhalten, in welchem das Wasser in dem Wassereingangssystem mittels Entgasungskammer 35 zum Entgasen des Wassers 11 und/oder mittels Luftabscheidekammer 23 zum Abscheiden von Luft, welche sich in dem Wasser befindet, entgast wird.

Das Verfahren kann in einer erfindungsgemäßen Ausführungsform den optionalen Schritt S13 beinhalten, wobei das Wasser 11 aus dem Zulauf 9 in einem vorangehenden Schritt in einem Fluidreservoir 7 gespeichert wird, wobei aus dem Fluidreservoir 7 mittels eines Überlaufs in den Behälter 1 zum Bestimmen des Füllstands des Wassers 11 fließt oder gefördert wird.

Alternativ zu Schritt 13 kann das erfindungsgemäße Verfahren in einer weiteren Ausführungsform den optionalen Schritt S15 beinhalten. Im Schritt 15 wird Wasser 11 aus dem Zulauf 9 zunächst im Fluidreservoir 7 gespeichert, anschließend fließt das Wasser 11 in den Heizbehälter 3 und daran anschließend mittels eines Überlaufs aus dem Heizbehälter 3 in den Behälter 1.

Das Verfahren kann in einer erfindungsgemäßen Ausführungsform den optionalen Schritt S17 beinhalten, in dem Wasser 11 zusätzlich mittels eines Wärmetauschers 17 erwärmt wird, wobei der Wärmetauscher 17 von Dialysat aus der Bilanzkammer 19 durchströmt wird.

### Bezugszeichenliste

- 100: Vorrichtung
- 200: Blutbehandlungsvorrichtung
- H1: vorbestimmter Füllstandswert
- S1 - S20: Verfahrensschritte
- 1: Behälter
- 3: Heizbehälter
- 5: Heizvorrichtung
- 7: Reservoir; Fluidreservoir
- 9: Zulauf; externer Zulauf
- 11: Fluid; Wasser
- 131,132,132,133, 134, 135: Ventil; Absperrventile
- 15: Entgasungsdrossel
- 17: Wärmetauscher
- 19: Bilanzkammer
- 21: Überlaufrichtung
- 23: Luftabscheider; Luftabscheidekammer
- 25: Pumpe im Vorlauf
- 27: mechanischer Füllstandsmesser; mechanischer Sensor; Schwimmer
- 29: vertikale Verschieberichtung des Schwimmers
- 31: Messeinrichtung
- 33: Steuervorrichtung
- 35: Entgasungskammer
- 360, 361, 362, 363, 364: Leitung; Verbindungsleitung
- 37: Pumpe im Dialysatkreislauf
- 39: Anschluss
- 41: Druckbegrenzungsventil

## Patentansprüche

1. Verfahren zum Regeln und/oder Überwachen einer Heizvorrichtung (5) zum Erwärmen eines über einen Zulauf (9) in einen Vorlauf eines Dialysierflüssigkeitskreislaufs zugeflossenen und/oder zufließenden Fluids (11), wobei der Dialysierflüssigkeitskreislauf Teil einer Blutbehandlungsvorrichtung (200) ist, wobei der Dialysierflüssigkeitskreislauf einen Behälter (1) zur Aufnahme des Fluids (11) und einen Heizbehälter (3) zum Erwärmen des Fluids (11) aufweist, wobei das Verfahren den Schritt umfasst:
- Starten eines Heizvorgangs zum Erwärmen des Fluids (11) im Heizbehälter (3), welcher in Fluidkommunikation mit dem Behälter (1) steht, nur dann, wenn der Füllstand des Behälters (1), insbesondere mittels direkten oder indirekten Zuflusses über den Zulauf (9), einen vorbestimmten Füllstandswert (H1) wenigstens einmal erreicht hat.

2. Verfahren nach Anspruch 1, wobei der Heizvorgang erst dann gestartet wird, wenn der Füllstand den vorbestimmten Füllstandswert (H1) mehrfach, insbesondere zweimal, dreimal, viermal oder fünfmal, erreicht hat.

3. Verfahren nach einem der vorangegangenen Ansprüche, wobei das Starten des Heizvorgangs zum Erwärmen des Fluids (11) im Heizbehälter (3) nur dann beginnt, nachdem ein Zufluss in den Vorlauf und/oder ein Ablauf aus dem Behälter (1) unterbunden gewesen waren oder nicht erfolgten.

4. Verfahren nach einem der vorangegangenen Ansprüche, wobei das Erreichen des vorbestimmten Füllstandswerts (H1) im Behälter (1) mittels eines mechanischen Füllstandsmessers (27) und/oder eines Leitfähigkeitssensors und/oder eines optischen Sensors bestimmt wird.

5. Verfahren nach einem der vorangegangenen Ansprüche, wobei das Fluid (11) aus dem Zulauf (9) in einem Fluidreservoir (7) gespeichert wird, das Fluid (11) anschließend in den Heizbehälter (3) fließt und daran anschließend mittels eines Überlaufs aus dem Heizbehälter (3) in den Behälter (1) fließt.

6. Verfahren nach einem der vorangegangenen Ansprüche, wobei das Fluid (11) zusätzlich mittels eines Wärmetauschers (17) erwärmt wird, wobei der Wärmetauscher (17) von Dialysat, insbesondere aus einer Bilanzkammer (19), durchströmt wird.

7. Verfahren nach einem der vorangegangenen Ansprüche, mit dem Schritt:
- Überwachen des Füllstands im Behälter (1), wobei der Heizvorgang angehalten oder gestoppt wird, wenn der Füllstand den vorbestimmten Füllstandswert (H1) unterschreitet.

8. Verfahren nach einem der vorangegangenen Ansprüche, wobei zum Starten des Heizvorgangs der Füllstand des Behälters (1) den vorbestimmten Füllstandswert (H1) über einen vorbestimmten Zeitraum T1 hinweg erreicht haben muss, insbesondere in einem Rezirkulationsmodus.

9. Verfahren nach einem der vorangegangenen Ansprüche, wobei wenigstens einer der folgenden Schritte mittels einer Steuervorrichtung (33) gesteuert oder geregelt wird:
- Starten oder Beenden des Heizvorgangs im Heizbehälter (3);
- Überwachen des Füllstands im Behälter (1); und
- Öffnen oder Schließen eines Absperrventils (131) zum Befüllen des Fluidreservoirs (7) und/oder des Behälters (1) aus dem Zulauf (9).

10. Steuer- oder Regelvorrichtung (33), programmiert oder konfiguriert, um das Verfahren gemäß einem der vorangegangenen Ansprüche auszuführen oder zu bewirken.

11. Blutbehandlungsvorrichtung (200), aufweisend wenigstens eine Steuervorrichtung (33) gemäß Anspruch 10.

12. Blutbehandlungsvorrichtung (200) nach Anspruch 11, mit wenigstens einem Behälter (1) zum Befüllen mit einem Fluid (11) mit einer Einrichtung zum Erkennen, ob der vorbestimmte Füllstandswert (H1) des Behälters erreicht ist, einem mit dem Behälter (1) in Fluidverbindung stehenden Heizbehälter (3) mit einer Heizvorrichtung (5) zum Erwärmen des Fluids (11), einer Einrichtung zum Weiterleiten des Fluids (11) in einen Dialysierflüssigkeitskreislauf der Blutbehandlungsvorrichtung (200).

13. Blutbehandlungsvorrichtung (200) nach einem der Ansprüche 11 bis 12, wobei ein Reservoir (7) oder der Behälter (1) angeordnet sind zu ihrem Befüllen mit Fluid (11) aus einem Zulauf (9) und/oder die Blutbehandlungsvorrichtung (200) einen Wärmetauscher (17) und/oder wenigstens einen Anschluss (39) für eine Konzentratzugabe aufweist.

14. Digitales Speichermedium, insbesondere in Form einer Diskette, CD oder DVD oder EPROM, mit elektronisch auslesbaren Steuersignalen, konfiguriert, um derart mit einem programmierbaren Computersystem zusammenzuwirken, dass die maschinellen Schritte eines erfindungsgemäßen Verfahrens nach einem der Ansprüche 1 bis 9 veranlasst werden.

15. Computerprogramm-Produkt, als Signalwelle oder mit einem auf einem maschinenlesbaren Träger gespeicherten Programmcode, zur Veranlassung der maschinellen Schritte des erfindungsgemäßen Verfahrens gemäß einem der Ansprüche 1 bis 9, wenn das Computerprogramm-Produkt auf einem Rechner abläuft.

## Claims

1. A method for regulating and/or monitoring a heating apparatus (5) for heating a fluid (11) having flowed and/or flowing via an inlet (9) into an in-flow section of a dialysis fluid circuit,
the dialysis fluid circuit being part of a blood treatment apparatus (200),
the dialysis fluid circuit comprising a container (1) for receiving the fluid (11) and a heating container (3) for heating the fluid (11),
wherein the method comprises the step of:
- starting a heating process for heating the fluid (11) in the heating container (3) being in fluid communication with the container (1), only when the filling level of the container (1) has reached a pre-determined filling level value (H1) at least once, in particular by a direct or indirect inflow through the inlet (9).

2. The method according to claim 1, wherein the heating process is only started when the filling level has reached the pre-determined filling level value (H1) several times, in particular two, three, four or five times.

3. The method according to anyone of the preceding claims, wherein the starting of the heating process for heating the fluid (11) in the heating container (3) starts only after an inflow into the in-flow section and/or an outflow from the container (1) have been prevented or have not occurred.

4. The method according to anyone of the preceding claims, wherein the reaching of the pre-determined filling level value (H1) in the container (1) is determined by means of a mechanical filling level meter (27) and/or of a conductivity sensor and/or of an optical sensor.

5. The method according to anyone of the preceding claims, wherein the fluid (11) from the inlet (9) is stored in a fluid reservoir (7), the fluid (11) subsequently flows into the heating container (3) and afterwards flows out of the heating container (3) into the container (1) by means of an overflow.

6. The method according to anyone of the preceding claims, wherein the fluid (11) is additionally heated by means of a heat exchanger (17), the heat exchanger (17) being flowed through by dialysate, in particular from a balancing chamber (19).

7. The method according to anyone of the preceding claims, comprising the step of:
- monitoring the filling level in the container (1), the heating process being paused or stopped when the filling falls below the pre-determined filling level value (H1).

8. The method according to anyone of the preceding claims, wherein to start the heating process, the filling level of the container (1) must have reached the pre-determined filling level value (H1) over a pre-determined period of time T1, in particular in a recirculation mode.

9. The method according to anyone of the preceding claims, wherein at least one of the following steps is controlled or regulated by means of a control device (33):
- starting or terminating the heating process in the heating container (3);
- monitoring the filling level in the container (1); and
- opening or closing a shut-off valve (131) for filling the fluid reservoir (7) and/or the container (1) from the inlet (9).

10. A control or regulating device (33), programmed or configured to execute or effect the method according to anyone of the preceding claims.

11. A blood treatment apparatus (200), comprising at least one control device (33) according to claim 10.

12. The blood treatment apparatus (200) according to claim 11, comprising at least a container (1) intended to be filled with a fluid (11), said container having a device for detecting whether the pre-determined filling level value (H1) of the container has been reached, a heating container (3) in fluid communication with the container (1) having a heating apparatus (5) for heating the fluid (11), a device for forwarding the fluid (11) into a dialysis liquid circuit of the blood treatment apparatus (200).

13. The blood treatment apparatus (200) according to anyone of the claims 11 to 12, wherein a reservoir (7) or the container (1) is arranged to be filled with fluid (11) from an inlet (9) and/or the blood treatment apparatus (200) comprises a heat exchanger (17) and/or at least one connection (39) for adding concentrate.

14. A digital storage medium, in particular in the form of a floppy disk, CD or DVD or EPROM, comprising electronically readable control signals, configured to interact with a programmable computer system so as to initiate the machine-induced steps of an inventive method according to anyone of claims 1 to 9.

15. A computer program product, as a signal wave or comprising a program code stored on a machine-readable support, for initiating the machine-induced steps of the inventive method according to anyone of claims 1 to 9, when the computer program product is running on a computer.

## Revendications

1. Un procédé destiné à réguler et/ou surveiller un appareil de chauffe (5) afin de réchauffer un fluide (11) s'écoulant et/ou entrant via un conduit d'alimentation (9) dans un conduit préliminaire d'un circuit de fluide de dialyse, le circuit de fluide de dialyse faisant partie d'un appareil de traitement du sang (200),
le circuit de fluide de dialyse comprenant un récipient (1) pour recevoir le fluide (11) ainsi qu'un récipient chauffant (3) pour réchauffer le fluide (11),
où le procédé comprend l'étape suivante consistant à:
- démarrer un processus de chauffe destiné à réchauffer le fluide (11) dans le récipient chauffant (3) étant en communication fluidique avec le récipient (1), uniquement si le niveau de remplissage du récipient (1) a atteint au moins une fois une valeur du niveau de remplissage prédéterminée (H1), notamment via un afflux direct ou indirect par le conduit d'alimentation (9).

2. Le procédé selon la première revendication, où le processus de chauffe n'est déclenché que lorsque le niveau de remplissage a atteint la valeur du niveau de remplissage prédéterminée (H1) à plusieurs reprises, notamment deux, trois, quatre ou cinq fois.

3. Le procédé selon l'une quelconque des revendications précédentes, où le démarrage du processus de chauffe destiné à réchauffer le fluide (11) dans le récipient chauffant (3) ne commence que lorsqu'un afflux dans le conduit préliminaire et/ou un écoulement hors du récipient (1) a/ont été empêché (s) ou ne se soit/soient pas produit(s).

4. Le procédé selon l'une quelconque des revendications précédentes, où l'atteinte de la valeur du niveau de remplissage prédéterminée (H1) dans le récipient (1) est déterminée au moyen d'une jauge du niveau de remplissage mécanique (27) et/ou d'un capteur de conductivité et/ou d'un capteur optique.

5. Le procédé selon l'une quelconque des revendications précédentes, où le fluide (11) provenant du conduit d'alimentation (9) est stocké dans un réservoir de fluide (7), le fluide (11) s'écoulant ensuite dans le récipient chauffant (3), puis s'écoulant du récipient chauffant (3) vers le récipient (1) au moyen d'un déversoir.

6. Le procédé selon l'une quelconque des revendications précédentes, où le fluide (11) est en outre réchauffé au moyen d'un échangeur de chaleur (17), l'échangeur de chaleur (17) étant traversé par du dialysat, provenant notamment d'une chambre d'équilibrage (19).

7. Le procédé selon l'une quelconque des revendications précédentes, comprenant l'étape suivante consistant à:
- surveiller le niveau de remplissage dans le récipient (1), le processus de chauffe étant interrompu ou arrêté lorsque le niveau de remplissage tombe en dessous de la valeur du niveau de remplissage prédéterminée (H1).

8. Le procédé selon l'une quelconque des revendications précédentes, où pour démarrer le processus de chauffe, le niveau de remplissage du récipient (1) doit avoir atteint la valeur du niveau de remplissage prédéterminée (H1) sur une certaine période de temps prédéterminée T1, notamment en mode de recirculation.

9. Le procédé selon l'une quelconque des revendications précédentes, où au moins l'une des étapes suivantes est commandée ou régulée au moyen d'un dispositif de commande (33):
- le démarrage ou l'arrêt du processus de chauffe dans le récipient chauffant (3);
- la surveillance du niveau de remplissage dans le récipient (1); et
- l'ouverture ou la fermeture d'une vanne d'arrêt (131) pour le remplissage du réservoir de fluide (7) et/ou du récipient (1) depuis le conduit préliminaire (9).

10. Un dispositif de commande ou de régulation (33), programmé ou configuré pour exécuter ou réaliser le procédé selon l'une quelconque des revendications précédentes.

11. Un appareil de traitement du sang (200), comprenant au moins un dispositif de commande (33) selon la revendication 10.

12. L'appareil de traitement du sang (200) selon la revendication 11, comprenant au moins un récipient (1) destiné à être rempli par un fluide (11), le récipient étant muni d'un dispositif permettant de distinguer si la valeur du niveau de remplissage prédéterminée (H1) du récipient est atteinte, un récipient chauffant (3) en communication fluidique avec le récipient (1) muni d'un appareil de chauffe (5) pour réchauffer le fluide (11), un dispositif pour transmettre le fluide (11) dans un circuit de liquide de dialyse de l'appareil de traitement du sang (200).

13. L'appareil de traitement du sang (200) selon l'une quelconque des revendications 11 à 12, où un réservoir (7) ou le récipient (1) est agencé pour être rempli de fluide (11) depuis un conduit préliminaire (9) et/ou l'appareil de traitement du sang (200) comprend un échangeur de chaleur (17) et/ou au moins un branchement (39) pour l'ajout de concentré.

14. Un support de stockage numérique, notamment sous la forme d'une disquette, d'un CD ou d'un DVD ou d'un EPROM, comportant des signaux de commande lisibles électroniquement, configuré pour interagir avec un système informatique programmable de manière à initier les étapes mécaniques d'un procédé conforme à l'invention selon l'une quelconque des revendications 1 à 9.

15. Un produit de programme informatique, sous forme d'onde de signal ou comprenant un code de programme stocké sur un support lisible par machine, pour initier les étapes mécaniques du procédé conforme à l'invention selon l'une quelconque des revendications 1 à 9, lorsque le produit de programme informatique est exécuté sur un ordinateur.
